# EUROPÄISCHE PATENTSCHRIFT

(11) **EP 3 160 526 B1**
(45) Veröffentlichungstag und Bekanntmachung des Hinweises auf die Patenterteilung: **18.07.2018**
(21) Anmeldenummer: 15729196.4
(22) Anmeldetag: 18.06.2015
(51) Int. Cl.: A61M 1/00, A61M 1/36

(54) **VORRICHTUNG ZUR ABSAUGUNG UND WEITERLEITUNG VON BLUT**
DEVICE FOR THE EXTRACTION AND FORWARDING OF BLOOD
DISPOSITIF D'ASPIRATION ET TRANSPORT DE SANG

(30) Priorität: 26.06.2014 EP 14174598
(43) Veröffentlichungstag der Anmeldung: 03.05.2017
(73) Patentinhaber: Medela Holding AG, 6340 Baar (CH)
(72) Erfinder: MANN, Robin, 78054 Villingen-Schwenningen (DE); WALTI, Martin, 8038 Zürich (CH)
(74) Vertreter: Rutz, Andrea
(86) Internationale Anmeldenummer: PCT/EP2015/063714
(87) Internationale Veröffentlichungsnummer: WO 2015/197463

(56) Entgegenhaltungen:
- EP-A1- 2 191 858
- EP-A1- 2 468 324
- WO-A1-98/20957
- US-A- 3 585 995
- US-A- 3 896 733
- US-A- 4 846 800
- US-B1- 8 361 042

## Beschreibung

### TECHNISCHES GEBIET

Die vorliegende Erfindung betrifft eine Vorrichtung zur Absaugung und zur Weiterleitung von Blut während einer chirurgischen Operation sowie einen Sekretsammelbehälter.

### STAND DER TECHNIK

Während oder nach chirurgischen Operationen, bei welchen der Patient einem starken Blutverlust ausgesetzt ist, ist es seit Jahren üblich, dem Patienten möglichst zeitnah Eigenblut zuzuführen. Bei der maschinellen Autotransfusion wird während der Operation abgesaugtes Blut extrakorporal gesammelt, üblicherweise gereinigt und dem Patienten wieder re-transfundiert. Insbesondere bei Verwendung einer Herz-Lungen-Maschine wird diese maschinelle Autotransfusion eingesetzt, um Blut aus der Operationswunde abzusaugen und ebenfalls dem Blutkreislauf des Patienten wieder zuzuführen.

Zum Transport des Bluts in dieser extrakorporalen Zirkulation werden üblicherweise peristaltische Pumpen eingesetzt. US 6 517 512 offenbart eine Rollenpumpe zur Verwendung mit einer Herz-Lungen-Maschine, wobei die Pumpe nach Massgabe eines Flüssigkeitsdetektorsignals automatisch an- und abschaltet. Andere Autotransfusionsgeräte mit Rollenpumpen sind beispielsweise aus US 3 799 702 oder EP 1 131 116 bekannt.

In US 2003/0144646 wird vorgeschlagen, einen Zwischenbehälter eines Autotransfusionsgeräts mittels Überdruck zu entleeren und einer Herz-Lungen-Maschine zuzuführen. Es wird vorgeschlagen, eine Rollenpumpe zur Erzeugung dieses Überdrucks zu verwenden.

Es ist bekannt, dass die empfindlichen Blutzellen bei der extrakorporalen Zirkulation häufig beschädigt werden und deshalb dem Patienten nicht mehr rückgeführt werden können. Beschädigte Zellen können zwar ausgefiltert werden. Dies ist jedoch aufwändig. Schlauch- oder Rollenpumpen beschädigen die Blutzellen mechanisch, da das Blut durch Quetschung des Schlauchs befördert wird. Bei starken Druckunterschieden lösen sich zudem die Zellmembranen der roten Blutkörperchen auf. In US 6 342 048 wird deshalb eine elektronisch gesteuerte Kolbenpumpe vorgeschlagen, deren Unterdruck entsprechend der in der Operationswunde anfallenden Menge abzusaugenden Bluts variiert wird. Die Reinfusion des gesammelten Blutes in den Kreislauf der Herz-Lungen-Maschine erfolgt nach wie vor über eine Rollenpumpe.

Da ein Patient über die Operationswunde mehrere Liter Blut verlieren kann, ist es notwendig, das abgesaugte Blut relativ schnell wieder in den Blutkreislauf zurückzubringen. Dabei darf die Wunde jedoch nicht mit einem zu grossen Unterdruck belastet werden. Wie bereits oben erwähnt, ist zudem eine schnelle Druckänderung beim Entleeren des Blutsammelbehälters zu vermeiden. US 3 896 733 schlägt deshalb vor, zwei Blutsammelbehälter zu verwenden, welche abwechslungsweise gefüllt und wieder entleert werden. Der Wechsel zwischen den zwei Behältern kann manuell oder mittels eines photoelektrischen Füllstandsensors automatisch erfolgen. Der eine Behälter wird mittels Unterdruck befüllt, während der andere Behälter belüftet wird, um das Blut möglichst rasch der Herz-Lungen-Maschine zuzuführen.

US 3 585 995 beschreibt einen Blutsammelbehälter mit zwei Kammern, deren Zu- und Ausgänge sich abwechslungsweise über Ventile steuern lassen.

Auch EP 2 080 529 offenbart ein kombiniertes Reservoir mit zwei voneinander getrennten Bereichen.

US 3 191 600 schlägt die Verwendung von zwei Blutsammelbehältern mit je zwei Kammern vor, wobei der Inhalt der oberen Kammern in die darunter liegende Kammer entleert wird.

US 4 846 800 beschreibt ein Autotransfusionsgerät mit einem Blutsammelbehälter, welcher zwei Kammern aufweist, die abwechslungsweise befüllt und entleert werden. Falls sich die Ausgänge der Kammern im unteren Bereich des Sammelbehälters befinden, sind Belüftungsventile vorgesehen, um den Unterdruck in der zu entleerenden Kammer aufzuheben. Sind die Ausgänge der Kammern im oberen Bereich des Sammelbehälters angeordnet, so werden die Kammern durch Anlegen eines Überdrucks entleert. Sind die Ausgänge oben angeordnet, so sind sie durch die oberen Enden von Steigrohren gebildet.

Weitere gattungsgemässe Vorrichtungen sind in der WO 98/20957, der EP 2 191 858, der US 8,361,042 und der EP 2 468 324 offenbart.

Obwohl Autotransfusion bereits seit vielen Jahren eingesetzt wird, fehlen Geräte, welche sich flexibel einsetzen lassen und welche auch in Notsituationen, d.h. wenn unerwartet viel Blut in der Operationswunde anfällt, optimal funktionieren. Zudem berücksichtigen die bekannten Geräte kaum die Gegebenheiten in einem Operationssaal. Die bekannten Autotransfusionsgeräte und insbesondere ihre Blutsammelbehälter müssen möglichst nahe beim Reservoir der Herz-Lungen-Maschine angeordnet sein, wobei die Blutsammelbehälter oberhalb dieses Reservoirs angeordnet sein müssen.

### DARSTELLUNG DER ERFINDUNG

Es ist deshalb eine Aufgabe der Erfindung, eine Vorrichtung zur Absaugung und zur Weiterleitung von Blut während einer chirurgischen Operation zu schaffen, welche eine blutschonende, jedoch effiziente und flexible Absaugung und Weiterleitung von Blut ermöglicht.

Diese Aufgabe löst eine Vorrichtung mit den Merkmalen des Patentanspruchs 1.

Die erfindungsgemässe Vorrichtung zur Absaugung und zur Weiterleitung von Blut während einer chirurgischen Operation weist eine erste Kammer und eine zweite Kammer zum Sammeln und Weiterleiten des abgesaugten Bluts sowie eine elektronisch gesteuerte Ventileinheit zur Verbindung der Kammern mit einer Vakuumquelle auf. Die Ventileinheit erstellt diese Verbindung abwechslungsweise zwischen der ersten Kammer und der Vakuumquelle und zwischen der zweiten Kammer und der Vakuumquelle, so dass die mit der Vakuumquelle verbundene erste oder zweite Kammer mit abgesaugtem Blut befüllbar und die andere zweite oder erste Kammer gleichzeitig entleerbar ist. Die Ventileinheit ist mit der ersten und der zweiten Kammer verbunden und ermöglicht eine Druckerhöhung in der zu entleerenden ersten oder zweiten Kammer. Die Druckerhöhung ist dank der Ventileinheit elektronisch verursacht und/oder elektronisch gesteuert.

Die Ventileinheit kann beispielsweise ein Vierwegventil umfassen, welches sowohl die Verbindung zur Vakuumquelle schafft wie auch die Druckerhöhung ermöglicht. Erfindungsgemäss weist die Ventileinheit aber ein erstes Ventilmodul auf, welches die Verbindung zwischen den Kammern und der Vakuumquelle schafft, und ein zweites Ventilmodul, welches die Druckerhöhung ermöglicht.

Die erfindungsgemässe Vorrichtung kann beispielsweise ein Autotransfusionsgerät zur Rückführung von Eigenblut in den Patienten sein oder sie kann ein Teil eines derartigen Geräts sein. Sie kann insbesondere Teil einer Herz-Lungen-Maschine sein bzw. in den extrakorporalen Kreislauf einer derartigen Maschine integriert sein.

Die erfindungsgemässe Vorrichtung weist den Vorteil auf, dass dank der Vakuumquelle eine schonende Absaugung des Blutes gewährleistet ist. Die steuerbare Ventileinheit gewährleistet, dass die zu entleerende Kammer rasch und effizient entleert werden kann.

Die Steuerung der zwei Ventilmodule erfolgt unabhängig voneinander, so dass auch eine Kammer befüllt werden kann, ohne dass die andere während der gesamten Befüllungszeit entleert werden muss, bzw. eine Kammer entleert werden kann, ohne dass die andere Kammer während der gesamten Entleerungszeit befüllt werden muss. Dies ist vorteilhaft, da das Blut in der Operationswunde oft in zeitlich variierender Menge anfällt. Die Unabhängigkeit der Steuerung der zwei Ventilmodule kann durch Sicherheitsmassnahmen durchbrochen sein.

Vorzugsweise ist die Ventileinheit mit einer Überdruckquelle verbindbar, wobei sie eine abwechslungsweise Verbindung zwischen der ersten Kammer und der Überdruckquelle und der zweiten Kammer und der Überdruckquelle schafft. Dadurch ist die zu entleerende erste oder zweite Kammer mit einem Druck höher als Atmosphärendruck entleerbar. Dies hat den Vorteil, dass die Entleerung beschleunigt ist. Sie kann so durchaus weniger lang dauern als die Befüllung. Dies ist insbesondere dann vorteilhaft, wenn die Herz-Lungen-Maschine entweder einen erhöhten Blutbedarf aufweist und/oder wenn eine relativ hohe Blutmenge in der Operationswunde zum Absaugen vorliegt.

Die Verwendung einer Überdruckquelle hat ferner den Vorteil, dass die erfindungsgemässe Vorrichtung unabhängig vom Ort der Herz-Lungen-Maschine im Operationssaal angeordnet werden kann. Ihr Blutsammelbehälter kann auch tiefer liegen als das Reservoir der Herz-Lungen-Maschine. Des Weiteren können die Schlauchverbindungen zum Patienten sowie zum Reservoir der Herz-Lungen-Maschine relativ lang sein. Dank des anliegenden Überdrucks ist die rasche und vollständige Entleerung der Kammer stets gewährleistet. Dies erlaubt auch, ununterbrochen Blut vom Patienten abzuführen.

Vorzugsweise sind ein erster Füllstandsensor zur Messung des Füllstandes in der ersten Kammer und ein zweiter Füllstandsensor zur Messung des Füllstandes in der zweiten Kammer vorhanden, wobei die Ventileinheit, insbesondere die zwei Ventilmodule, nach Massgabe mindestens eines dieser Sensorsignale gesteuert sind. Dies ermöglicht eine vollautomatische Steuerung der Ventileinheit und insbesondere der zwei Ventilmodule.

Vorzugsweise ist das Gerät als kompaktes Gerät ausgebildet und somit platzsparend im Operationssaal. Hierzu ist die Ventileinheit, insbesondere das erste und zweite Ventilmodul, in einem Gehäuse angeordnet. Vorzugsweise sind die erste und die zweite Kammer lösbar an diesem Gehäuse fixiert gehalten. Die Kompaktheit und Bedienbarkeit der Vorrichtung ist erhöht, wenn die erste und zweite Kammer in einem gemeinsamen Blutsammelbehälter angeordnet sind, welcher lösbar am Gehäuse fixiert gehalten ist. Vorzugsweise sind auch die Vakuumquelle und/oder die Überdruckquelle im Gehäuse angeordnet. Vorzugsweise ist im Gehäuse ferner eine Steuereinheit zur Steuerung der Ventileinheit angeordnet.

In einer Ausführungsform sind die Vakuumquelle und die Überdruckquelle zwei separate Pumpen. In einer anderen Ausführungsform sind sie durch eine einzige Pumpe gebildet, beispielsweise indem der Auspuff der Vakuumpumpe als Überdruckquelle verwendet wird.

Die Vakuumquelle und/oder die Überdruckquelle sind nicht-peristaltische Pumpen, vorzugsweise sind sie Kolbenpumpen und noch bevorzugter Membranpumpen. Vorzugsweise sind sie regelbare Pumpen, insbesondere elektromotorisch betriebene Pumpen.

Es lassen sich auch mehr als zwei Kammern verwenden, wobei dann die Ventileinheit vorzugsweise der Reihe nach die erste, zwei und dritte bzw. nachfolgende Kammer bedient.

In einer bevorzugten Ausfuhrungsform sind die Kammern so ausgebildet, dass sie auch bei einem geringen Füllstand bereits eine zuverlässige Messung ermöglichen. Dies lässt sich beispielsweise dadurch erreichen, dass ihr Innenraum einen nach unten verjüngenden Querschnitt aufweist. Als Füllstandsensoren lassen sich bekannte Sensoren, z.B. optische oder optoelektronische Sensoren verwenden. Wird ein kapazitiver Sensor verwendet, so erstreckt er sich vorzugsweise von einem oberen Bereich der Kammer nach unten bis in den sich verjüngenden Bereich hinein.

Derartige Sammelkammern lassen sich auch in anderen Bereichen und nicht nur für die Blutsammlung einsetzen. Ein entsprechender Behälter wird hiermit somit als zusätzliche Erfindung betrachtet. Beispielsweise lässt sich ein derartiger Behälter mit einer Kammer oder mit zwei oder mehr Kammern in der medizinischen Drainage, z.B. der Thoraxdrainage, Wunddrainage oder der Fettabsaugung, entsprechend verwenden. Der Behälter ist deshalb nachfolgend teilweise als Sekretsammelbehälter und nicht als Blutsammelbehälter bezeichnet, wobei die hier gegebene Beschreibung der Behälterbeschaffenheit jeweils auf beide Arten von Behältern zutrifft.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung eines Patienten mit einer ersten Herz-Lungen-Maschine und der erfindungsgemässen Vorrichtung;
- Figur 2: eine schematische Darstellung eines Patienten mit einer zweiten Herz-Lungen-Maschine und der erfindungsgemässen Vorrichtung;
- Figur 3: eine perspektivische Darstellung einer erfindungsgemässen Vorrichtung gemäss einer ersten Ausführungsform;
- Figur 4: eine schematische Darstellung des Inneren eines Gehäuses der Vorrichtung gemäss Figur 3;
- Figur 5: eine perspektivische Darstellung einer erfindungsgemässen Vorrichtung gemäss einer zweiten Ausführungsform;
- Figur 6: einen Längsschnitt durch die Vorrichtung gemäss Figur 5;
- Figur 7: eine perspektivische Darstellung eines erfindungsgemässen Sekretsammelbehälters in einer ersten Ausführungsform, mit sichtbar dargestelltem Innenraum;
- Figur 8: einen ersten Längsschnitt durch den Sekretsammelbehälter gemäss Figur 7;
- Figur 9: einen zweiten Längsschnitt durch den Sekretsammelbehälter gemäss Figur 7;
- Figur 10: einen Querschnitt durch den Sekretsammelbehälter gemäss Figur 7;
- Figur 11: ein Schaltbild einer Variante der Vorrichtung gemäss Figur 3;
- Figur 12: ein Schaltbild der erfindungsgemässen Vorrichtung in einer zweiten Ausführungsform;
- Figur 13: ein Schaltbild der erfindungsgemässen Vorrichtung in einer dritten Ausführungsform und
- Figur 14: ein Schaltbild der erfindungsgemässen Vorrichtung in einer vierten Ausführungsform.

In den Figuren sind gleiche Teile mit gleichen Bezugszeichen versehen.

### BESCHREIBUNG BEVORZUGTER AUSFÜHRUNGSFORMEN

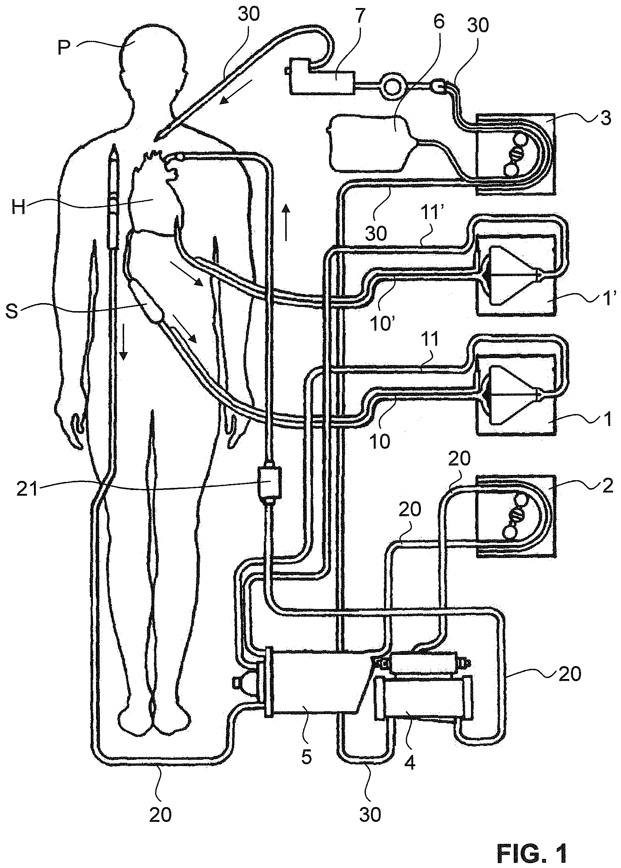

Figur 1 zeigt einen Patienten P mit einer extrakorporalen Blutzirkulation mittels einer Herz-Lungen-Maschine. Sein Herz ist in der Figur mit H bezeichnet, eine offene Operationswunde mit S. Die Strömungsrichtung des Blutes ist mit Pfeilen dargestellt.

Die Herz-Lungen-Maschine besteht im Wesentlichen aus
- einem Schlauchsystem 20, welches einen extrakorporalen Kreislauf bildet,
- einem im Schlauchsystem 20 befindlichen Reservoir 5, in welchem das Blut gesammelt wird,
- einer im Schlauchsystem 20 dem Reservoir 5 nachfolgenden Zentrifugalpumpe oder ersten peristaltischen Pumpe 2, welche auf einen Bereich des Schlauchsystems 20 einwirkt und so das darin befindliche Blut transportiert,
- einen im Schlauchsystem 20 der Pumpe 2 nachgeschalteten Oxygenator 4, welcher das Blut mit einer genügenden Sauerstoffsättigung versorgt, bevor es anschliessend über ein oder mehrere Filter 21 dem Gefässsystem des Patienten P rückgeführt wird.

Wird das Herz H des Patienten P stillgelegt, so wird üblicherweise eine zweite peristaltische Pumpe 3 mit einer Zusatzleitung 30 verwendet. Diese zweite Pumpe 3 führt dem Patienten P über einen Wärmetauscher 7 Blut vom Oxygenator 4 zu. Dabei wird dem Blut eine kardioplegische Lösung aus einem Lösungsbehälter 6 der Herz-Lungen-Maschine beigemengt.

Als Herz-Lungen-Maschine und für die Stilllegung des Herzens können auch andere bekannte Systeme eingesetzt und mit der nachfolgend beschriebenen erfindungsgemässen Vorrichtung verwendet werden. Zudem kann die nachfolgend beschriebene erfindungsgemässe Vorrichtung auch ohne Herz-Lungen-Maschine eingesetzt werden. In diesem Fall weist sie üblicherweise allfällige zusätzliche Elemente zur Wiederaufbereitung des Blutes vor der Rückführung in den menschlichen Körper auf.

Nachfolgend wird die erfindungsgemässe Vorrichtung beschrieben:
Das in der Operationswunde S anfallende Blut wird in diesem Beispiel dem Patienten P mittels Autotransfusion rückgeführt. Hierzu führt eine erste patientenseitige Saugleitung 10 zu einem ersten erfindungsgemässen Autotransfusionsgerät 1. Von diesem Gerät 1 führt eine erste Weiterleitung 11 zum Reservoir 5 der Herz-Lungen-Maschine.

Je nach Grösse der Operationswunde oder nach Art der Operation können weitere Autotransfusionsgeräte zum Einsatz kommen. In Figur 1 ist ein zweites erfindungsgemässes Gerät 1' mit entsprechender Saugleitung 10' vom Patienten P zum Gerät und einer Weiterleitung 11' vom Gerät 1' ins Reservoir 5 dargestellt.

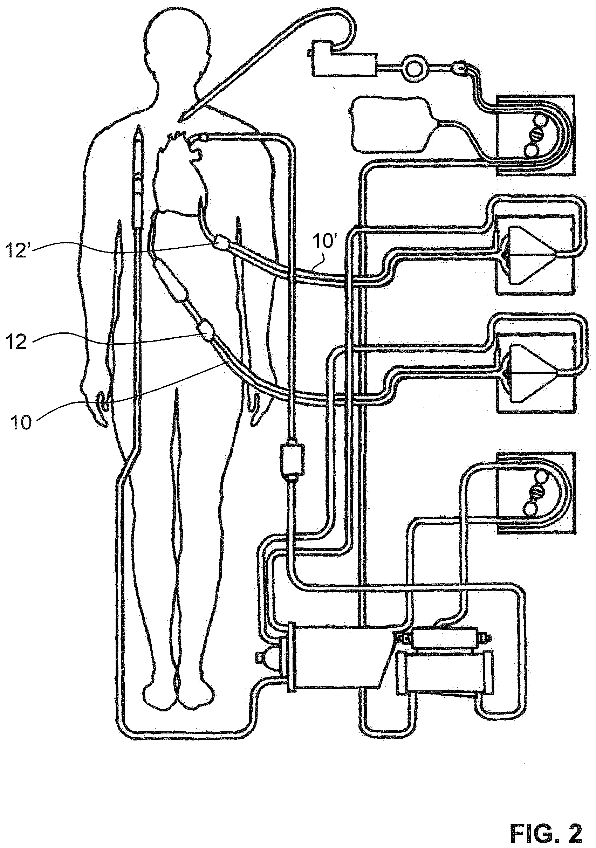

Das Ausführungsbeispiel gemäss Figur 2 ist praktisch mit demjenigen aus Figur 1 identisch, wobei hier nun in beiden patientenseitigen Saugschläuchen 10, 10' der Autotransfusionen je ein Überdruckventil 12, 12' zum Schutz des Patienten vorhanden ist.

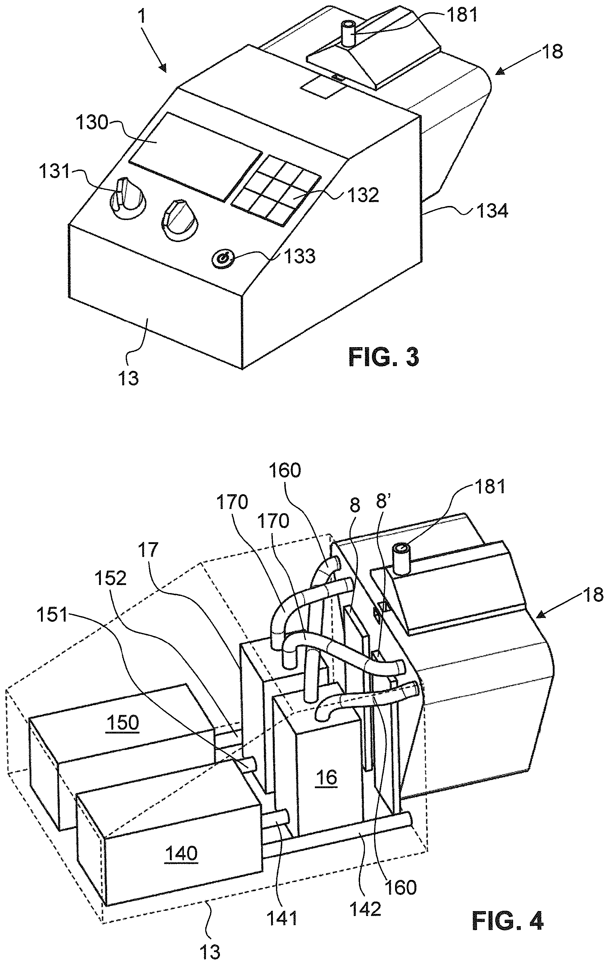

In Figur 3 ist ein erfindungsgemässes Autotransfusionsgerät 1 bzw. eine erfindungsgemässe Vorrichtung als Teil eines derartigen Geräts dargestellt. Es weist ein Gehäuse 13 und einen daran lösbar gehaltenen Blutsammelbehälter 18 auf.

Am Gehäuse 13 sind Anzeige- und Betätigungsmittel vorhanden, wie beispielsweise ein Display 130, Betätigungsknöpfe 131, Betätigungstasten 132 und eine Ein- und Ausschalttaste 133.

Der Blutsammelbehälter 18 ist vorzugsweise auf einer Seite, hier der Rückseite 134 des Gehäuses 13 angeordnet. Er weist einen patientenseitigen Anschluss 181 zur Verbindung mit der patientenseitigen Saugleitung 10 auf. Ferner ist ein weiterleitungsseitiger Anschluss zur Verbindung mit der Weiterleitung 11 vorhanden, welcher in Figur 3 nicht sichtbar ist, da er sich in diesem Ausführungsbeispiel auf der Unterseite des Behälters 18 befindet.

Im Gehäuse 13 befindet sich, wie in Figur 4 erkennbar ist, eine Vakuum- oder Unterdruckpumpe 140 mit einem zugehörigen ersten Ventilmodul 16, eine Überdruckpumpe 150 mit einem zugehörigen zweiten Ventilmodul 17 sowie diverse Leitungen. Die zwei Ventilmodule 16, 17 bilden eine Ventileinheit. Anstelle von zwei voneinander getrennten und unabhängigen Modulen lässt sich auch eine Ventileinheit mit einem einzigen Modul verwenden, indem dieses beispielsweise ein Vierwegventil ist.

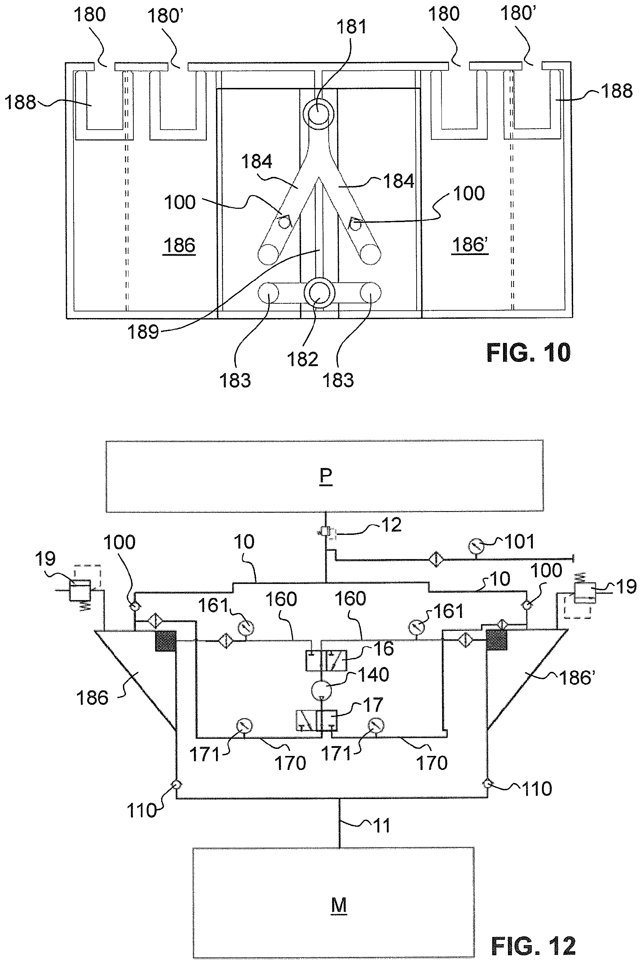

Eine erste Verbindungsleitung 141 verbindet die Vakuumpumpe 140 mit dem ersten Ventilmodul 16, eine Auspuffleitung 142 verbindet die Vakuumpumpe 140 mit der Aussenseite des Gehäuses 13. Von dem ersten Ventilmodul 16 führen zwei voneinander getrennte Vakuumleitungen 160 zu Auslassöffnungen in der Rückwand 134 des Gehäuses 13. Diese Auslassöffnungen sind dichtend mit ersten Anschlussöffnungen 180 (siehe Figur 10) des Blutsammelbehälters 18 verbindbar bzw. verbunden.

Eine zweite Verbindungsleitung 151 verbindet die Überdruckpumpe 150 mit dem zweiten Ventilmodul 17, eine Ansaugleitung 152 verbindet die Überdruckpumpe 150 mit der Aussenseite des Gehäuses 13. Vom zweiten Ventilmodul 17 führen zwei voneinander getrennte Überdruckleitungen 170 zu zweiten Auslassöffnungen in der Rückwand 134 des Gehäuses 13. Diese Auslassöffnungen sind dichtend mit zweiten Anschlussöffnungen 180' (siehe Figur 10) des Blutsammelbehälters 18 verbindbar bzw. verbunden.

An der Rückwand 134 des Gehäuses oder alternativ am Blutsammelbehälter 18 sind Füllstandsensoren 8, 8' angeordnet. Vorzugsweise sind es kapazitive Füllstandsensoren. Sie erstrecken sich vorzugsweise von der Unterseite des Behälters 18 bis zu einer maximal zulässigen Füllhöhe.

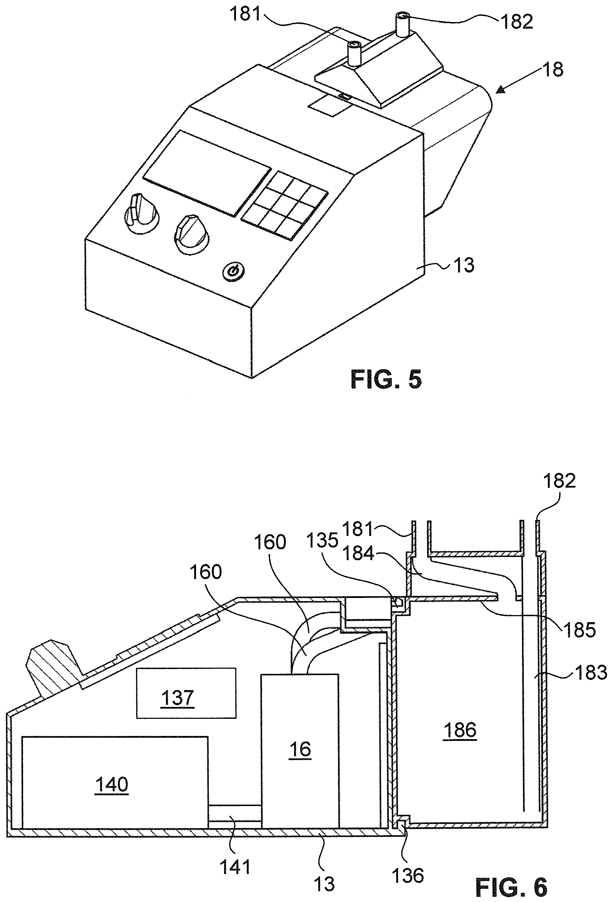

In den Figuren 5 und 6 ist eine zweite Ausfuhrungsform der erfindungsgemässen Vorrichtung dargestellt. Sie unterscheidet sich vom ersten Ausführungsbeispiel dadurch, dass der patientenseitige Anschluss 181 und der weiterleitungsseitige Anschluss 182 beide auf der Oberseite des Blutsammelbehälters 18 angeordnet sind. Der weiterleitungsseitige Anschluss 182 geht in ein Steigrohr oder eine Steigleitung 183 über, welche sich bis in den unteren Bereich einer Blut-Sammelkammer 186 erstreckt. Der patientenseitige Anschluss 181 ist ebenfalls mit dieser Kammer 186 verbunden, beispielsweise über einen Verbindungskanal oder einen Verbindungsschlauch 184. Vorzugsweise trennt ansonsten eine horizontal verlaufende Trennrippe den Bereich der Anschlüsse 181, 182 von der Kammer 186 und weist lediglich zwei Durchgangsöffnungen für die genannten Verbindungen auf. Dies verhindert ein Rückschwappen oder ein Zurückspritzen von Blut in die Saugleitung 10 oder in die Weiterleitung 11.

Ferner ist in Figur 6 eine Variante erkennbar, wie der Behälter 18 am Gehäuse 13 lösbar gehalten sein kann. In diesem Beispiel ist er einrastbar, wobei auf der oberen und unteren Seite des Behälters 18 und des Gehäuses 13 gegenseitig in Eingriff bringbare Einrastelemente 135, 136 vorhanden sind.

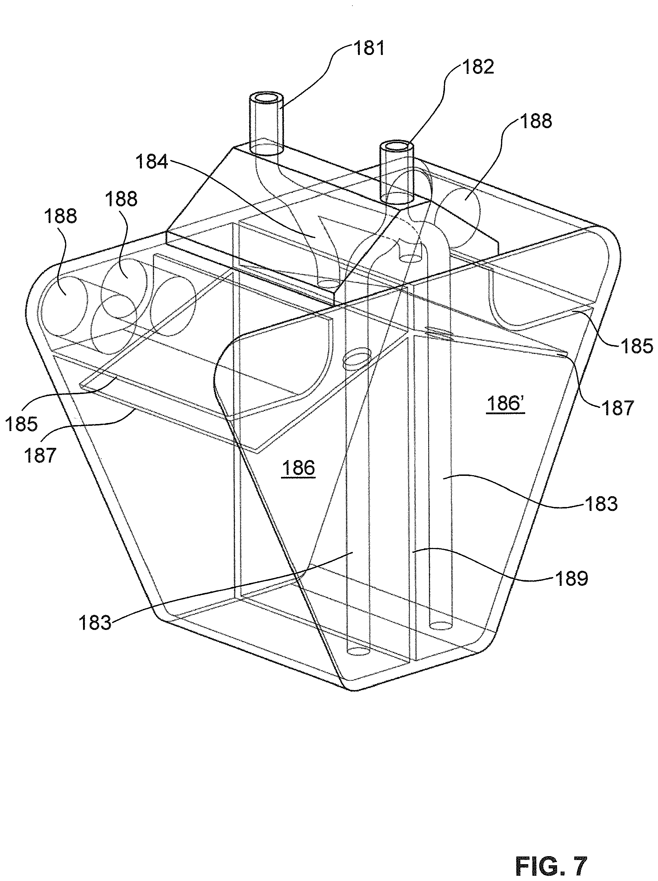

In den Figuren 7 bis 10 ist eine bevorzugte Ausfuhrungsform eines erfindungsgemässen Blutsammelbehälters 18 mit zwei Kammern 186, 186' dargestellt. Der Behälter 18 ist vorzugsweise einstückig ausgebildet. Vorzugsweise besteht er aus einem Kunststoff. Vorzugsweise ist er durchsichtig. Er ist vorzugsweise als Einwegprodukt ausgebildet und wird nach erfolgter chirurgischer Operation entsorgt.

Der Behälter 18 weist auf seiner Oberseite den patientenseitigen Anschluss 181 und den weiterleitungsseitigen Anschluss 182 auf. Der Behälter 18 ist ansonsten spiegelsymmetrisch aufgebaut. Vom patientenseitigen Anschluss 181 führt die sich verzweigende, eine Y-Form aufweisende Verbindungsleitung 184 in den Behälterinnenraum. Vom weiterleitungsseitigen Anschluss 182 führt eine Y-Verzweigung in zwei Steigrohre 183, welche sich bis in den unteren Bereich des Innenraums des Behälters erstrecken.

Der Innenraum des Behälters 18 ist in zwei Bereiche unterteilt, welche vorzugsweise vollständig voneinander getrennt sind. Sie befinden sich in diesem Beispiel nebeneinander und sind durch eine Trennwand 189 voneinander getrennt. Je eine der genannten Verbindungsleitungen 184 und je eines der genannten Steigrohre 183 führen in je einen der Bereiche. Im Folgenden wird ein Bereich beschrieben. Der andere Bereich ist identisch aufgebaut.

Der Bereich wird im Wesentlichen durch eine Blutsammelkammer 186 gebildet, welche in ihrem oberen Bereich von einer Schrägrippe 187 begrenzt ist. Diese Schrägrippe 187 erstreckt sich von der Trennwand 189 beginnend zur gegenüberliegenden Seite hin nach unten, wobei sie sich fast vollständig, jedoch nicht ganz vollständig über die gesamte Breite der Kammer erstreckt. In der dazu senkrechten Richtung erstreckt sich die Schrägrippe 187 über die gesamte Länge des Bereichs bzw. der Kammer 186. Die Schrägrippe 187 ist vom Steigrohr 183 durchsetzt.

Im Bereich oberhalb der Schrägrippe 187 ist die sich über die gesamte Länge des Bereichs hin erstreckende und nach oben gebogene Trennrippe 185 angeordnet, welche einen pumpenseitigen Bereich definiert. Dieser Bereich ist lediglich durch einen schmalen oberen Spalt mit dem restlichen Innenraum verbunden. In diesem Bereich sind die erste und zweite Anschlussöffnung 180, 180' angeordnet, welche zur Verbindung mit der Vakuumleitung 160 bzw. der Überdruckleitung 170 dienen. Diese Anschlussöffnungen 180, 180' sind mit Filter 188 versehen. Diese Filter sind im Stand der Technik bekannt. Es können auch noch andere Filter an anderen Stellen vorhanden sein, wie dies ebenfalls aus dem Stand der Technik bekannt ist. Diese Filter dienen zum Schutz des Geräts.

Die Trennrippe 185 verhindert, dass Blut zu den Filtern spritzen kann und diese vorzeitig verschliessen kann. Zudem bildet sie einen weiteren Schutz für die Pumpen. Die Schrägrippe 187 verhindert ein Zurückschwappen des abgesaugten Bluts in die Saugleitung und ermöglicht zudem, dass abgesaugtes Blut über die Schrägrippe 187 nach unten in die Sammelkammer 186 fliessen kann.

Die Kammer 186 weist einen sich nach unten verjüngenden Querschnitt auf. Vorzugsweise beträgt die Fläche im untersten Bereich der Kammer 186 maximal die Hälfte der Fläche im oberen Bereich, d.h. im Bereich knapp unterhalb des unteren Endes der Schrägrippe 187. Dies ermöglicht eine relativ genaue Füllstandmessung selbst bei geringen Füllmengen, insbesondere wenn hierfür kapazitive Füllstandsensoren eingesetzt werden.

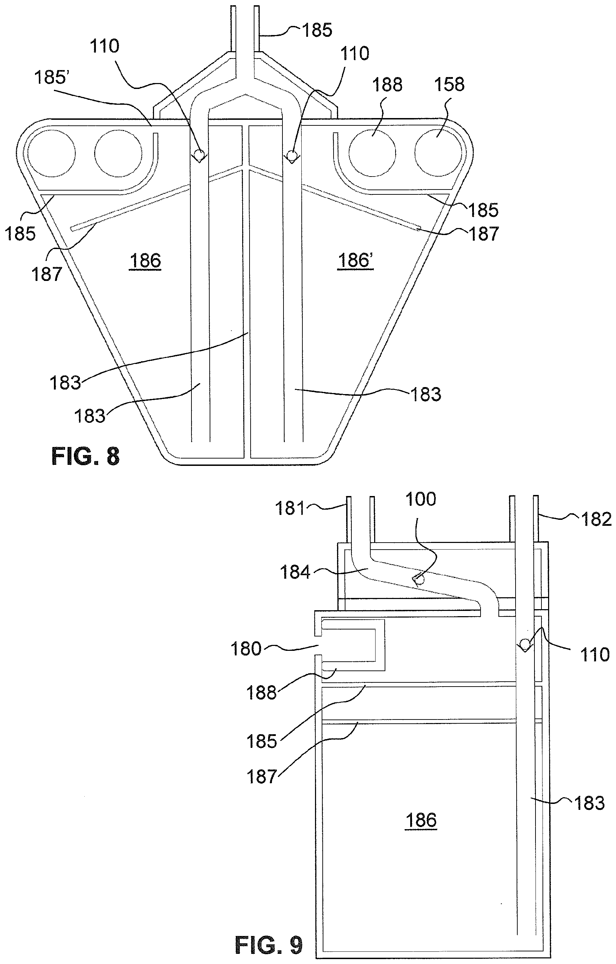

Vorzugsweise weist der Blutsammelbehälter 18 deshalb eine rechteckige Bodenfläche, jedoch zwei einander gegenüberliegende trapezförmige Seitenwände sowie zwei diese trapezförmigen Seitenwände verbindende Schrägwände und eine im Wesentlichen rechteckförmige obere Wand auf, wie dies in den Figuren 7 und 8 gut erkennbar ist. Vorzugsweise sind alle Wände im Wesentlichen plan ausgebildet. Vorzugsweise sind lediglich die zwei einander gegenüberliegenden Seitenwände schräg ausgebildet und die übrigen Wände verlaufen in vertikaler oder horizontaler Richtung.

Die zwei Kammern 186 des Behälters 18 lassen sich abwechslungsweise befüllen und entleeren. D.h. wird eine Kammer befüllt, so lässt sich die andere gleichzeitig entleeren. Dabei wird spätestens dann entleert, wenn der entsprechende Füllstandsensor 8, 8' einer Steuereinheit 137 des Geräts 1 meldet, dass die entsprechende Kammer 186 voll ist.

Entsprechend sind Einwegventile 100 vorhanden, welche die Verbindung zwischen der Saugleitung 10 und der Sammelkammer 186 öffnen und verschliessen. Diese können über die Steuerung gesteuert sein oder bei einem vorgegebenen Druck schliessen. Sie können, wie in den Figur 9 und 10 dargestellt, Teil des Blutsammelbehälters 18 sein oder sie können auch im Bereich der Saugleitung 10 bzw. beim patientenseitigen Anschluss 181 angeordnet sein.

Es sind ferner Einwegventile 110 vorhanden, welche die Verbindung zwischen der Sammelkammer 86 und der Weiterleitung 11 öffnen und verschliessen. Diese können ebenfalls über die Steuerung gesteuert sein oder bei einem vorgegebenen Druck schliessen. Sie können, wie in den Figuren 8 und 9 dargestellt, Teil des Blutsammelbehälters 18 sein oder sie können auch im Bereich der Weiterleitung 11 bzw. beim weiterleitungsseitigen Anschluss 182 angeordnet sein.

Die Einwegventile können beispielsweise einfache Rückschlagventile sein oder sie können ebenfalls über die Steuereinheit 137 gesteuert sein.

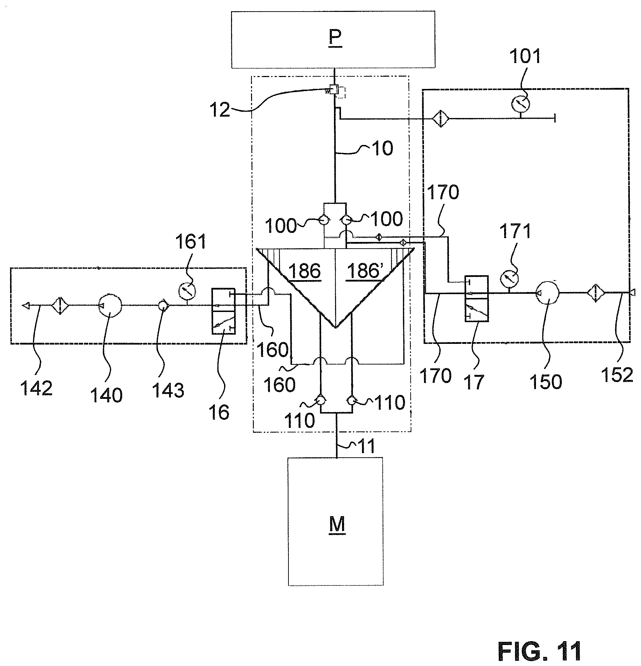

Anhand Figur 11 wird nun die Funktionsweise dieses Geräts 1 beschrieben. Der Patient P ist an eine Herz-Lungen-Maschine M angeschlossen, wobei ferner eine Autotransfusion mittels des erfindungsgemässen Geräts stattfindet.

Die Vakuumpumpe 140 ist über das erste Ventilmodul 16 wahlweise mit einer der beiden Sammelkammern 186 verbindbar. Ist sie mit der linken Kammer 186 verbunden, so ist ihre Verbindung zur rechten Kammer 186 unterbrochen, wie dies hier dargestellt ist. In der linken Kammer 186 wird somit ein Unterdruck erzeugt und das Blut wird bei geöffnetem linken patientenseitigen Ventil 100 in die linke Kammer 186 abgesaugt. Dabei ist das linke weiterleitungsseitige Ventil 110 verschlossen.

Die rechte Kammer 186, welche mit Blut gefüllt ist, lässt sich gleichzeitig entleeren und das gesammelte Blut der Herz-Lungen-Maschine über die Weiterleitung 11 zuführen. Hierzu ist die rechte Kammer 186 mittels des zweiten Ventilmoduls 17 mit der Überdruckpumpe 150 verbunden, deren Verbindung zur linken Kammer 186 gleichzeitig unterbrochen ist. In die rechte Kammer 186 wird bei geschlossenem rechten patientenseitigen Ventil 100 ein Überdruck, d.h. ein Druck höher als Atmosphärendruck angelegt, wobei das Blut dank geöffnetem rechten weiterleitungsseitigen Ventil 110 abfliessen kann.

Eine Beeinträchtigung des Patienten wird zusätzlich vermieden, indem vorzugsweise ein Überdruckventil 12 im Saugschlauch 10 vorhanden ist. Vorzugsweise überwacht zudem ein Drucksensor 101 den Druck in der Saugleitung. Dieser Drucksensor 101 ist vorzugsweise ebenfalls Bestandteil des erfindungsgemässen Geräts 1 und er ist vorzugsweise mit der Steuerung des Geräts 1, insbesondere mit der Steuerung der Überdruckpumpe 150 verbunden. Hierzu wird vorzugsweise ein doppellumiger Schlauch verwendet, wobei ein Lumen als Saugleitung und das zweite Lumen als Messleitung dient. Ein derartiger Schlauch ist in den Figuren 1 und 2 dargestellt. Derartige Systeme sind aus dem medizinischen Drainagebereich bekannt. Die Messleitung kann alternativ oder zusätzlich als Spülleitung dienen, um den Schlauch von Verstopfungen zu befreien oder um eine medizinische Lösung dem Patienten zuzuführen.

Ist die linke Kammer 186 gefüllt, so wird gewechselt: d.h. die rechte Kammer 186 wird nun mit Unterdruck beaufschlagt und die linke Kammer dank Überdruck entleert.

Die Vakuumpumpe 140 und die Überdruckpumpe 150 sind nicht-peristaltische Pumpen. Vorzugsweise sind sie Kolbenpumpen und noch bevorzugter Vakuummembranpumpen. Diese lassen sich sehr gezielt steuern bzw. regeln. Das Rückschlagventil 143 dient zur Erhaltung des Vakuums, auch wenn die Vakuumpumpe 140 zeitweise ausgeschaltet ist. Vorzugsweise sind Drucksensoren bzw. Manometer 161, 171 vorhanden, um den in der jeweiligen Pumpe erzeugten Druck zu überwachen und je nach Ausfuhrungsform auch zu steuern.

In Figur 12 ist eine Variante des erfindungsgemässen Geräts dargestellt. Hier dient eine einzige Pumpe 140 sowohl als Vakuumpumpe wie auch als Überdruckpumpe. Die Funktion als Überdruckpumpe wird erreicht, indem der Auspuff als Überdruckleitung verwendet wird und dieser deshalb an das zweite Ventilmodul 17 angeschlossen ist. In diesem Beispiel sind die zwei Kammern 186 ferner mit Überdruckventilen 19 versehen, welche bei einem zu hohen Überdruck in der jeweiligen Kammer 186 öffnen. Diese Überdruckventile 19 lassen sich auch in den übrigen Ausführungsbeispielen verwenden.

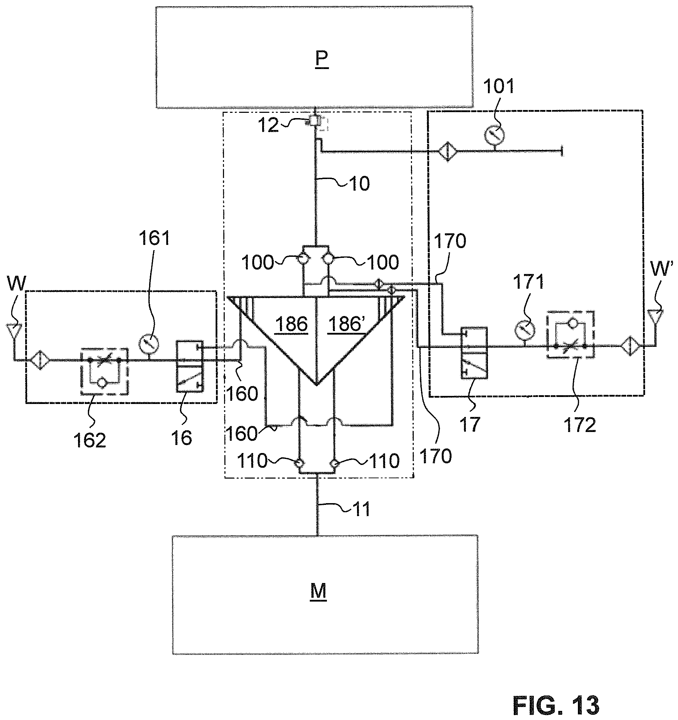

In der Variante gemäss Figur 13 verfügt das Gerät 1 selber über keine Pumpen, sondern es lässt sich mit Wandanschlüssen W, W' des Spitals verbinden, um so an eine Vakuumquelle und an eine Überdruckquelle angeschlossen zu werden. Es sind entsprechende Eingangsventile 162, 172 vorhanden, um die Verbindung mit der entsprechenden Saugquelle zu öffnen und zu schliessen.

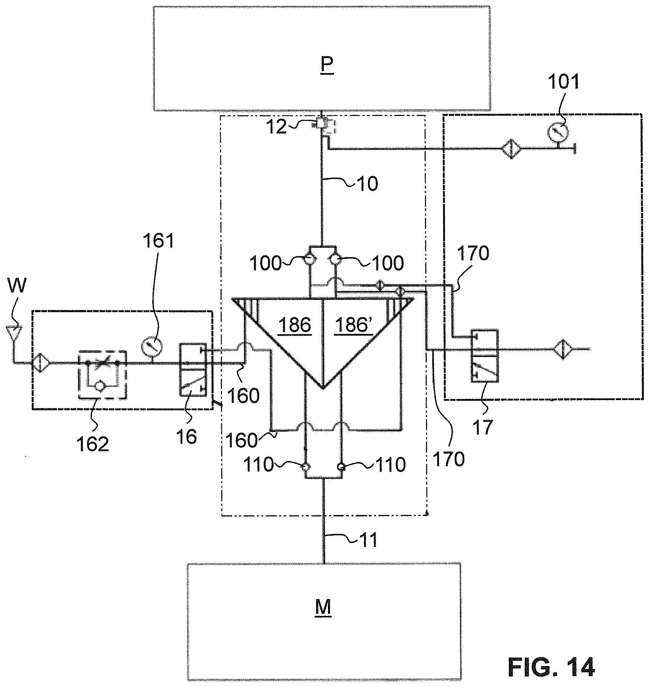

In der Ausfuhrungsform gemäss Figur 14 ist nur ein Wandanschluss W für die Vakuumquelle vorhanden. Auf der Entleerungsseite wird lediglich belüftet, d.h. die befüllte Kammer 186 wird auf Atmosphärendruck gebracht, um entleert zu werden.

Das erfindungsgemässe Gerät ermöglicht eine blutschonende, aber effiziente Sammlung und Weiterleitung von Blut während Operationen. Der erfindungsgemässe Sekretsammelbehälter ermöglicht eine erhöhte Genauigkeit in der Detektion des Füllstandes.

**BEZUGSZEICHENLISTE**

| | | | |
|---|---|---|---|
| 1 | erstes Autotransfusionsgerät | 151 | zweite Verbindungsleitung |
| 10 | erste patientenseitige Saugleitung | 152 | Ansaugleitung |
| | | | |
| 100 | patientenseitiges Ventil | 16 | erstes Ventilmodul |
| 101 | dritter Drucksensor | 160 | Vakuumleitung |
| 11 | erste Weiterleitung | 161 | erster Drucksensor |
| 110 | weiterleitungsseitiges Ventil | 162 | Eingangsventil |
| 12 | erstes Überdruckventil | | |
| | | 17 | zweites Ventilmodul |
| 1' | zweites Autotransfusionsgerät | 170 | Überdruckleitung |
| 10' | zweite patientenseitige Saugleitung | 171 | zweiter Drucksensor |
| | | 172 | Eingangsventil |
| 11' | zweite Weiterleitung | | |
| 12' | zweites Überdruckventil | 18 | Blutsammelbehälter |
| | | 180 | erste Anschlussöffnung |
| 13 | Pumpengehäuse | 180' | zweite Anschlussöffnung |
| 130 | Display | 181 | patientenseitiger Anschluss |
| 131 | Betätigungsknopf | 182 | weiterleitungsseitiger Anschluss |
| 132 | Betätigungstasten | | |
| 133 | Ein/Ausschalttaste | 183 | Steigleitung |
| 134 | Rückwand | 184 | Verbindungsleitung |
| 135 | oberes Einrastelement | 185 | Trennrippe |
| 136 | unteres Einrastelement | 185' | Spalt |
| 137 | Steuereinheit | 186 | erste Sammelkammer |
| | | 186' | zweite Sammelkammer |
| 140 | Vakuumpumpe | 187 | Schrägrippe |
| 141 | erste Verbindungsleitung | 188 | Filter |
| 142 | Auspuffleitung | 189 | Trennwand |
| 143 | Rückschlagventil | | |
| | | 19 | Überdruckventil |
| 150 | Überdruckpumpe | | |
| 2 | erste peristaltische Pumpe | 7 | Wärmetauscher |
| 20 | Schlauchsystem des extrakorporalen Kreislaufs | | |
| | | 8 | erster Füllstandsensor |
| 21 | Filter | 8' | zweiter Füllstandsensor |
| | | | |
| 3 | zweite peristaltische Pumpe | H | Herz |
| 30 | Zusatzleitung | S | Operationsort |
| | | P | Patient |
| 4 | Oxygenator | M | Herz-Lungen-Maschine |
| | | W | Wandanschluss für Unterdruck |
| 5 | Reservoir | | |
| | | W' | Wandanschluss für |
| 6 | Lösungsbehälter | | Überdruck |

## Patentansprüche

1. Vorrichtung zur Absaugung und zur Weiterleitung von Blut während einer chirurgischen Operation,
wobei die Vorrichtung eine erste Kammer (186) und eine zweite Kammer (186') zum Sammeln und Weiterleiten des abgesaugten Bluts sowie eine Ventileinheit (16, 17) zur Verbindung der Kammern (186, 186') mit einer Vakuumquelle (140, W) aufweist,
wobei die Ventileinheit (16, 17) diese Verbindung abwechslungsweise zwischen der ersten Kammer (186) und der Vakuumquelle (140, W) und zwischen der zweiten Kammer (186') und der Vakuumquelle (140, W) erstellt, so dass die mit der Vakuumquelle (140, W) verbundene erste oder zweite Kammer (186, 186') mit abgesaugtem Blut befüllbar und die andere zweite oder erste Kammer (186', 186) gleichzeitig entleerbar ist,
und wobei die Ventileinheit (16, 17) mit der ersten und der zweiten Kammer (186, 186') verbunden ist und eine Druckerhöhung in der zu entleerenden ersten oder zweiten Kammer (186, 186') ermöglicht,
**dadurch gekennzeichnet, dass** die Ventileinheit (16, 17) elektronisch gesteuert ist und ein erstes Ventilmodul (16) aufweist, welches die Verbindung zwischen der ersten und zweiten Kammer (186, 186') und der Vakuumquelle (140, W) erstellt, sowie ein zusätzliches zweites Ventilmodul (17) aufweist, welches die Druckerhöhung ermöglicht, und dass das erste und das zweite Ventilmodul (16, 17) unabhängig voneinander steuerbar sind.

2. Vorrichtung nach Anspruch 1, wobei die Ventileinheit (16, 17) mit einer Überdruckquelle (150, W') verbindbar ist und eine abwechslungsweise Verbindung zwischen der ersten Kammer (186) und der Überdruckquelle (150, W) und der zweiten Kammer (186') und der Überdruckquelle (150, W') schafft, so dass die zu entleerende erste oder zweite Kammer (186, 186') mit einen Druck höher als Atmosphärendruck entleerbar ist.

3. Vorrichtung nach einem der Ansprüche 1 oder 2, wobei ein erster Füllstandsensor (8) zur Messung des Füllstandes in der ersten Kammer (186) und ein zweiter Füllstandsensor (8') zur Messung des Füllstandes in der zweiten Kammer (186') vorhanden ist, wobei die Ventileinheit (16, 17) nach Massgabe eines Sensorsignals mindestens eines dieser Füllstandsensoren (8, 8') gesteuert sind.

4. Vorrichtung nach einem der Ansprüche 1 bis 3, wobei die Ventileinheit (16, 17) in einem Gehäuse (13) angeordnet ist, wobei die erste und die zweite Kammer (186, 186') lösbar am Gehäuse (13) fixiert gehalten sind.

5. Vorrichtung nach Anspruch 4, wobei die erste und zweite Kammer (186, 186') in einem gemeinsamen Blutsammelbehälter (18) angeordnet sind, welcher lösbar am Gehäuse (13) fixiert gehalten ist.

6. Vorrichtung nach einem der Ansprüche 4 bis 5, wobei die Vakuumquelle (140, W) und/oder die Überdruckquelle (150, W') im Gehäuse (13) angeordnet sind.

7. Vorrichtung nach einem der Ansprüche 4 bis 6, wobei im Gehäuse (13) eine Steuereinheit (137) zur Steuerung der Ventileinheit (16, 17) angeordnet ist.

8. Vorrichtung nach einem der Ansprüche 1 bis 6 wobei die Vakuumquelle (140) und die Überdruckquelle (150) zwei separate Pumpen sind oder wobei die Vakuumquelle und die Überdruckquelle durch eine einzige Pumpe (140) gebildet sind.

9. Vorrichtung nach einem der Ansprüche 1 bis 8, wobei die Vakuumquelle und/oder die Überdruckquelle nicht-peristaltische Pumpen (140, 150), vorzugsweise Kolben- oder Membranpumpen, sind.

10. Vorrichtung nach einem der Ansprüche 1 bis 8, wobei die die Vakuumquelle und/oder die Überdruckquelle regelbare Pumpen (140, 150) sind.

11. Vorrichtung nach einem der Ansprüche 1 bis 10, wobei die Kammern (186, 186') einen sich nach unten hin verjüngenden Innenquerschnitt aufweisen.

12. Vorrichtung nach Anspruch 3, wobei sich der Füllstandsensor (8, 8') jeder Kammer (186, 186') von oben nach unten bis in den Bereich des sich verjüngenden Innenquerschnitts hin erstreckt.

13. Sekretsammelbehälter, insbesondere ein Blutsammelbehälter (18) zur Verwendung in einer Vorrichtung gemäss einem der Ansprüche 1 bis 12, wobei der Sekretsammelbehälter einen Eingang (181) und einen Ausgang (182) und mindestens einen Innenraum (186, 186') aufweist, wobei der Innenraum (186, 186') durch eine Trennwand (189) in zwei Bereiche unterteilt ist, die im Wesentlichen jeweils durch eine Blutsammelkammer gebildet werden, und wobei der Innenraum (186, 186') einen sich nach unten verjüngenden Innenquerschnitt aufweist, **dadurch gekennzeichnet, dass** beide Blutsammelkammern in ihrem oberen Bereich jeweils von einer Schrägrippe (187) begrenzt sind.

## Claims

1. Device for aspirating and transferring blood during a surgical operation
wherein the device has a first chamber (186) and a second chamber (186') for collecting and transferring the aspirated blood, and a valve unit (16, 17) for connecting the chambers (186, 186') to a vacuum source (140, W),
wherein the valve unit (16, 17) establishes this connection alternately between the first chamber (186) and the vacuum source (140, W) and between the second chamber (186') and the vacuum source (140, W), such that the first or second chamber (186, 186') connected to the vacuum source (140, W) can be filled with aspirated blood, and the other, second or first chamber (186', 186) can be emptied at the same time,
and wherein the valve unit (16, 17) is connected to the first and second chamber (186, 186') and permits a pressure increase in the first or second chamber (186, 186') to be emptied,
**characterized in that** the valve unit (16, 17) is electronically controlled and has a first valve module (16) which establishes the connection between the first and second chamber (186, 186') and the vacuum source (140, W), and has an additional second valve module (17), which permits the pressure increase, and **in that** the first and second valve modules (16, 17) are controllable independently of each other.

2. Device according to Claim 1, wherein the valve unit (16, 17) is connectable to an overpressure source (150, W') and creates an alternate connection between the first chamber (186) and the overpressure source (150, W) and the second chamber (186') and the overpressure source (150, W'), such that the first or second chamber (186, 186') to be emptied can be emptied with a pressure higher than atmospheric pressure.

3. Device according to one of Claims 1 or 2, wherein a first filling level sensor (8) for measuring the filling level in the first chamber (186) and a second filling level sensor (8') for measuring the filling level in the second chamber (186') are present, wherein the valve unit (16, 17) is controlled according to a sensor signal from at least one of these filling level sensors (8, 8').

4. Device according to one of Claims 1 to 3, wherein the valve unit (16, 17) is arranged in a housing (13), wherein the first and second chambers (186, 186') are held releasably in a fixed position on the housing (13).

5. Device according to Claim 4, wherein the first and second chambers (186, 186') are arranged in a common blood collection container (18), which is held releasably in a fixed position on the housing (13) .

6. Device according to one of Claims 4 to 5, wherein the vacuum source (140, W) and/or the overpressure source (150, W') are arranged in the housing (13).

7. Device according to one of Claims 4 to 6, wherein a control unit (137) for controlling the valve unit (16, 17) is arranged in the housing (13).

8. Device according to one of Claims 1 to 6, wherein the vacuum source (140) and the overpressure source (150) are two separate pumps, or wherein the vacuum source and the overpressure source are formed by a single pump (140).

9. Device according to one of Claims 1 to 8, wherein the vacuum source and/or the overpressure source are non-peristaltic pumps (140, 150), preferably piston pumps or diaphragm pumps.

10. Device according to one of Claims 1 to 8, wherein the vacuum source and/or the overpressure source are controllable pumps (140, 145).

11. Device according to one of Claims 1 to 10, wherein the chambers (186, 186') have a downwardly tapering internal cross section.

12. Device according to Claim 3, wherein the filling level sensor (8, 8') of each chamber (186, 186') extends from the top downward into the area of the tapering internal cross section.

13. Secretion collection container, in particular a blood collection container (18) for use in a device according to one of Claims 1 to 12, wherein the secretion collection container has an inlet (181) and an outlet (182) and at least one interior (186, 186'), wherein the interior (186, 186') is divided by a separating wall (189) into two areas which are basically formed by a blood collection chamber in each case, and wherein the interior (186, 186') has a downwardly tapering internal cross section, **characterized in that** both blood collection chambers are delimited in their upper area by an oblique rib (187) in each case.

## Revendications

1. Un dispositif pour l'aspiration et le transfert de sang pendant une opération chirurgicale, où le dispositif comporte une première chambre (186) et une seconde chambre (186') pour collecter et transférer le sang aspiré, ainsi qu'un unité de vannes (16, 17) pour une liaison des chambres (186, 186') à une source de vide (140, W),
où l'unité de vannes (16, 17) établit cette liaison de manière alternée entre la première chambre (186) et la source de vide (140, W) et entre la seconde chambre (186') et la source de vide (140, W), de telle sorte que la première ou seconde chambre (186, 186') qui est reliée à la source de vide (140, W) peut être remplie par le sang aspiré et que l'autre seconde ou première chambre (186', 186) peut être vidée en même temps,
et où l'unité de vannes (16, 17) est reliée à la première et la seconde chambre (186, 186') et permet d'augmenter la pression dans la première ou la seconde chambre (186, 186') à vider,
**caractérisé en ce que** l'unité de vannes (16, 17) est commandée de manière électronique et comporte une premier module de vanne (16), lequel établit une liaison entre la première et seconde chambre (186, 186') et la source de vide (140, W), ainsi qu'un second module de vanne (17) supplémentaire, lequel permet d'augmenter la pression, et que le premier ou le second module de vanne (16, 17) peuvent être commandés de manière indépendante l'une par rapport à l'autre.

2. Le dispositif selon la revendication 1, où l'unité de vannes (16, 17) peut être reliée à une source de pression (150, W') et établit cette liaison de manière alternée entre la première chambre (186) et la source de pression (150, W) et ente la seconde chambre (186 ') et la source de pression (150, W'), de telle sorte que la première ou seconde chambre (186, 186') à vider peut être vidée à l'aide d'une pression supérieure à la pression atmosphérique.

3. Le dispositif selon une des revendications 1 ou 2, où un premier capteur de niveau (8) pour la mesure du niveau dans la première chambre (186) et un second capteur de niveau (8') pour la mesure du niveau dans la seconde chambre (186') est présent, où l'unité de vannes (16, 17) est commandée dans la mesure d'un signal de capteur d'au moins un des capteurs de niveau (8, 8').

4. Le dispositif selon une des revendications 1 à 3, où l'unité de vannes (16, 17) est disposée dans un boitier (13), où la première et seconde chambre (186, 186') sont maintenues sur le boîtier (13) de manière amovible.

5. Le dispositif selon la revendication 4, où la première et seconde chambre (186, 186') sont disposées dans un récipient de collecte de sang commun (18), lequel est maintenu sur le boîtier (13) de manière amovible.

6. Le dispositif selon une des revendications 4 à 5, où la source de vide (140, W) et / ou la source de pression (150, W') sont disposées dans le boîtier (13).

7. Le dispositif selon une des revendications 4 à 6, où une unité de commande (137) est disposée dans le boîtier (13) pour commander l'unité de vannes (16, 17).

8. Le dispositif selon une des revendications 1 à 6, où la source de vide (140) et la source de pression (150) sont deux pompes séparées, ou où la source de vide et la source de pression sont formées par une seule pompe (140).

9. Le dispositif selon une des revendications 1 à 8, où la source de vide et / ou la source de pression sont des pompes non-péristaltiques (140, 150), préférablement des pompes à piston ou membrane.

10. Le dispositif selon une des revendications 1 à 8, où la source de vide et / ou la source de pression sont des pompes réglables (140, 145).

11. Le dispositif selon une des revendications 1 à 10, où les chambres (186, 186') présentent une section transversale intérieure diminuante vers le bas.

12. Le dispositif selon la revendication 3, où le capteur de niveau (8, 8') de chaque chambre (186, 186') s'étend depuis le haut vers le bas jusque dans le domaine de la section transversale intérieure diminuante.

13. Un récipient de collecte de sécrétions, en particulier un récipient de collecte de sang (18) pour l'utilisation dans un dispositif selon une des revendications 1 à 12, où le récipient de collecte de sécrétions comporte une entrée (181) et une sortie (182) et au moins un espace intérieur (186, 186'), où l'espace intérieur (186, 186') est subdivisé en deux domaines par une paroi de séparation (189), qui sont sensiblement formées respectivement par une chambre de collecte de sang, et où l'espace intérieur (186, 186') présente une section transversale intérieure diminuant vers le bas, **caractérisé en ce que** les deux chambres de collecte de sang sont limitées dans leur domaines supérieurs respectivement par une nervure oblique (187).
